# EUROPEAN PATENT SPECIFICATION

(11) **EP 1 061 060 B1**
(45) Date of publication and mention of the grant of the patent: **12.01.2005**
(21) Application number: 99906479.3
(22) Date of filing: 25.02.1999
(51) Int. Cl.: C07C 31/22, C07C 29/147, C07C 29/76, C07B 53/00

(54) **PROCESS FOR PRODUCING 1,2,4-BUTANETRIOL**
VERFAHREN ZUR HERSTELLUNG VON 1,2,4-BUTANTRIOL
PROCEDE DE PRODUCTION DE 1,2,4-BUTANETRIOL

(30) Priority: 03.03.1998 JP 5072398
(43) Date of publication of application: 20.12.2000
(73) Proprietor: DAISO CO., LTD., Osaka-shi, Osaka 550-0002 (JP)
(72) Inventor: IKAI, Kousei, Amagasaki-shi, Hyogo 660-0842 (JP); MIKAMI, Masafumi, Amagasaki-shi, Hyogo 660-0842 (JP); FURUKAWA, Yoshiro, Amagasaki-shi, Hyogo 660-0842 (JP); HO, Shohin, Amagasaki-shi, Hyogo 660-0842 (JP)
(74) Representative: Peaucelle, Chantal
(86) International application number: PCT/JP1999/000867
(87) International publication number: WO 1999/044976

(56) References cited:
- WO-A-99/23086
- WO-A1-98/08793
- JP-A- 1 068 366
- PAWLAK J ET AL: "STEREOCHEMICAL STUDIES OF POLYOLS FROM THE POLYENE MACROLIDE LIENOMYCIN" JOURNAL OF ORGANIC CHEMISTRY,US,AMERICAN CHEMICAL SOCIETY. EASTON, vol. 52, no. 13, 26 June 1987 (1987-06-26), pages 2896-2901, XP002047203 ISSN: 0022-3263
- THIAM M. ET AL.: "Malic Acid as Chiral Synthon : Synthesis of 1,2 and 1,3 Optically Active Diols" SYNTHETIC COMMUNICATIONS, vol. 22, no. 1, 1992, pages 83-95, XP000984435
- SHIBATA TOMOYUKI et al., "Synthesis of Optically Active 3-Mercaptopyrrolidine Derivatives. Synthetic Intermediates of Carbapenem RS-533 and its Isomer", HETEROCYCLES, 1986, Vol. 24, No. 5, p. 1331-1346, XP002920960

## Description

### Technical Field

The present invention relates to a process for preparing 1, 2, 4-butanetriol, particularly optically active 1, 2, 4-butanetriol, which is a useful compound as a synthetic intermediate of medicines, agricultural chemicals and the like.

### Background Art

1, 2, 4-Butanetriol is an industrially useful compound which has been known from old times, and it has been used as a synthetic precursor of 3-hydroxytetrahydrofuran and 3-hydroxypyrrolidine derivatives and the like. There have been reported three conventional processes for preparing this compound by the following processes: 1) a process for reducing dimethyl malate with lithium aluminum hydride (J. Amer. Chem. Soc., 103, 9, 2273-2280 (1981)), 2) a process for reducing dimethyl malate with sodium borohydride in a mixed solvent of THF/H₂O (J. Org. Chem., 52, 13, 2896-2901 (1987)) and 3) a process for reducing malic acid with a borane-dimethyl sulfide complex in THF (Tetrahedron Lett., 26, 42, 5195-5198 (1985)).

However, the above-mentioned conventional processes had the following problems. First, in the process 1), lithium aluminum hydride, which is difficult to handle industrially and expensive, has to be used as a reducing agent. When an optically active substance is used as the starting material, it is difficult to obtain a target product having a high optical purity, since racemization proceeds during the reaction (J. Org. Chem., 48, 16, 2767-2769 (1983)). Accordingly, it is necessary to carry out troublesome steps wherein a hydroxyl group is protected and then ester groups are reduced to give an optically active target product through deprotection, or a reduced product is converted into its derivative and then the derivative is recrystallized to raise its optical purity. In the process 2), since THF is used as the solvent, it is feared that THF produces a peroxide and the peroxide explodes when THF is concentrated. Accordingly, this process is difficult to apply to industry. In addition, since drain is contaminated by THF to give wastewater and wastewater treatment is necessary; this process has many problems in practice. In the process 3), since dimethyl sulfide gives out a bad smell and it is expensive, it is industrially difficult to use the borane-dimethyl sulfide complex. Since THF is used as the solvent, the above-mentioned fear and wastewater problems are unavoidable.

In Synth.Comm., 22(1), pp 83-95, the authors disclose the reduction of dimethyl malate by two equivalents of borane-dimethyl sulfide and by sodium borohydride. But the reaction solvent is THF which may result in the production of peroxide and explosion risks.

WO 99/23086 discloses a process for the preparation of 1, 2, 4-butanetriol by reduction of hydroxybutane dioic acid dimethyl ester with an alkali metal borohydride. But THF is also used in said process.

WO 98/08793 discloses a process for the preparation of 1,2,4-butanetriol by reduction of a malic acid diester in a solvent mixture comprising an ether and an alcohol. The ester is dissolved in the alcohol, which is preferably ethanol, and the solution is added to a mixture of NaBH₄ in the ether.

For these reasons, an economically inexpensive process for obtaining 1, 2, 4-butanetriol, which is a useful compound as a synthetic intermediate of medicines, agricultural chemicals and the like, by using an easily available compound as a starting material has been desired.

An object of the present invention is to provide a process wherein 1, 2, 4-butanetriol can be obtained safely, easily and inexpensively without causing the problems concerning wastewater.

### Disclosure of the Invention

Studying in order to solve the above-mentioned problems, the present inventors found that desired 1, 2, 4-butanetriol can be prepared inexpensively by using a malic diester, 3-hydroxy- γ -butyrolactone or a 3, 4-dihydroxybutanoate as a starting compound and by reducing this compound with a specific reducing agent, and completed the present invention.

The present inventors also found that substantially no racemization occurs during the reduction and optically active 1, 2, 4-butanetriol is obtained when an optically active substance is used as the above-mentioned starting compound.

The present invention relates to a process for preparing 1, 2, 4-butanetriol characterized in that a malic diester, 3-hydroxy-γ-butyrolactone or a 3, 4-dihydroxybutanoate represented by the following formulae (I), or a mixture thereof is reduced with sodium borohydride in an alcohol selected from the group consisting of methanol, ethanol, propanol, isopropanol, ethylene glycol, propylene glycol and a mixture thereof or a mixed solvent composed of an alcohol selected from the group consisting of methanol, ethanol, propanol, isopropanol, ethylene glycol, propylene glycol, and of aromatic solvent(s) to give 1, 2, 4-butanetriol represented by the following formula (II): 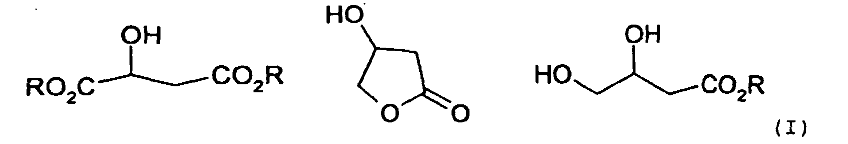 (wherein "R" is an alkyl group having four carbon atoms or fewer).

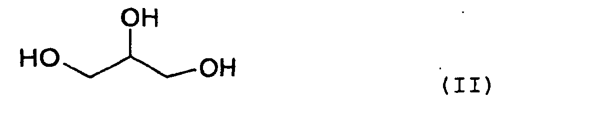

When the malic diester, 3-hydroxy- γ -butyrolactone and the 3, 4-dihydroxybutanoate to be used as the starting materials of the process are racemates (I), the obtained 1, 2,4-butanetriol is also a racemate (II). The above-mentioned three starting compounds can be used individually or in combination.

The malic diester, 3-hydroxy-γ-butyrolactone or the 3,4-dihydroxybutanoate to be used as the starting material can be compound obtained by any methods. The malate can be obtained, for example, according to Tetrahedron Lett. 33, 11, 1415-1418 (1992). 3-Hydroxy- γ - butyrolactone and the 3, 4-dihydroxybutanoate can be obtained, for example, by the method described in Japanese Laid-open Patent Publication No. 47296/1997.

The alkyl group R of the malic diester or the 3, 4-dihydroxybutanoate is not limited to particular groups so far as the alkyl group has four carbon atoms or fewer. Preferred alkyl group is a methyl group or an ethyl group in terms of reactivity of the reduction.

The malic diester, 3-hydroxy- γ -butyrolactone and the 3, 4-dihydroxybutanoate to be used as the starting materials of the process can be optically active substances represented by the following formulae (III). In this case, substantially no recemization occurs during the reduction, and the obtained 1, 2, 4-butanetriol is an optically active substance represented by the following formula (IV). In this reaction, the above-mentioned three optically active starting compounds can also be used individually or in combination, and optically active 1, 2, 4-butanetriol is obtained in either case. 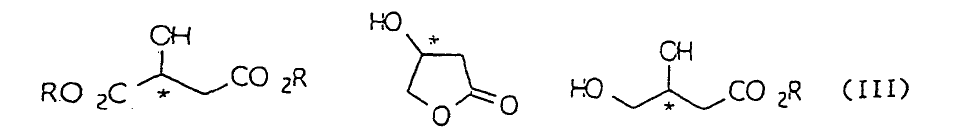 (wherein "R" is an alkyl group having four carbon atoms or fewer)

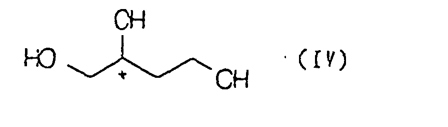

When the optically active substance is prepared, the optically active malic diester, optically active 3-hydroxy- γ-butyrolactone and the optically active 3, 4-dihydroxybutanoate which are the starting materials can also be obtained by the methods described in the above-mentioned literatures. The alkyl group R of the optically active malic diester or 3, 4-dihydroxybutanoate can also be a group having four carbon atoms or fewer. Preferred alkyl group is a methyl group or an ethyl group in terms of reactivity of the reduction.

In the process of the invention, preferred organic solvents are alcohols or mixed solvents containing an alcohol. Preferred alcohols are aliphatic alcohols having four carbon atoms or fewer in terms of solubility of sodium borohydride. Examples of the alcohol are methanol, ethanol, propanol, isopropanol, ethylene glycol and propylene glycol. Methanol, ethanol and ethylene glycol are more preferable among them. Methanol and ethanol are the most preferable in terms of yield. These alcohols can be used individually or in combination.

The solvent can be the mixed solvent mainly composed of the alcohol. In the case of this mixed solvent, examples of other solvents combined with the alcohol are aromatic solvents such as benzene, toluene and xylene; hydrocarbon solvents such as hexane, heptane and cyclohexane; and halogenic solvents such as dichloromethane, chloroform and 1, 2-dichloroethane.

Sodium borohydride to be used in said process can be a commercially available product. When the starting material is the optically active or racemic malic diester, an amount of sodium borohydride is preferably 60 to 200 g, more preferably 60 to 100 g to one mole of the malic diester. When the amount of the reducing agent is less than 60 g, the reaction is not completed, and the yield is liable to decrease. When the amount is more than 200 g, an amount of the solvent to dissolve the reducing agent increases, which is disadvantageous in terms of cost and yield. When the starting material is optically active or racemic 3-hydroxy-γ-butyrolactone or 3, 4-dihydroxybutanoate, the amount of the reducing agent is preferably 30 to 100 g, more preferably 30 to 50 g mole to one mole of 3-hydroxy- γ -butyrolactone or the 3, 4-dihydroxybutanoate. When the amount of the reducing agent is less than 30 g, the reaction is not completed, and the yield is liable to decrease. When the amount is more than 100 g, the amount of the solvent to dissolve the reducing agent increases, which is disadvantageous in terms of the cost and yield.

Examples of modes of said process are i) a method wherein sodium borohydride is added to a solution of the malic diester, 3-hydroxy- γ-butyrolactone, the 3, 4-dihydroxybutanoate or the mixture thereof, which is the starting material, in the alcohol or the mixed solvent containing the alcohol, ii) a method wherein sodium borohydride is suspended in a solvent which is inert on sodium borohydride, and the malic diester, 3-hydroxy-γ-butyrolactone, the 3, 4-dihydroxybutanoate or the mixture thereof, which is the starting material, and the alcohol or the mixed solvent containing the alcohol are added (preferably dropwise) thereto separately or in the form of a solution wherein they are mixed, iii) a method wherein sodium borohydride and the malic diester, 3-hydroxy- γ -butyrolactone, the 3, 4-dihydroxybutanoate or the mixture thereof, which is the starting material, are suspended in the solvent which is inert on sodium borohydride, and the alcohol or the mixed solvent containing the alcohol is added (preferably dropwise) thereto, and the like.

In the above-mentioned modes ii) and iii), the reaction can be carried out at a high concentration of sodium borohydride, so that the reaction is efficient, and these modes are more preferable. Furthermore, since the liquid is added dropwise to the suspension as mentioned above in the methods of these modes, operation is also simpler than that of the method wherein sodium borohydride, which is a solid, is added. An amount of the alcohol or the mixed solvent containing the alcohol to be used in these modes is preferably one to ten equivalents, more preferably one to five equivalents to sodium borohydride.

Examples of the organic solvent which is inert on sodium borohydride are aromatic solvents such as benzene, toluene and xylene; hydrocarbon solvents such as hexane, heptane and cyclohexane; and halogenic solvents such as dichloromethane, chloroform and 1, 2-dichloroethane.

Reaction temperature of the reduction by said process is not limited to a particular range, and the temperature is preferably -20° to 80°C , more preferably 0° to 50°C. When the temperature is lower than this range, the reaction proceeds slowly, viscosity of a reaction liquid also increases, and stirring is liable to be difficult. When the temperature exceeds this range, the solvent reacts with sodium borohydride, and the yield tends to decrease. The reaction is usually carried out under ordinary pressure and can be carried out under elevated pressure, if necessary. Reaction time is adjusted depending on reaction temperature and reaction pressure.

In the said process, the object product is obtained by post-treating a reaction mixture with a mineral acid after the reaction, filtering out the resulting insoluble matter, evaporating an excess solvent under reduced pressure and adding a base to the resulting residue to neutralize it, or by treating the reaction mixture with an ion exchange resin and then carrying out usual purification such as distillation. Hydrogen chloride or sulfuric acid is preferable in terms of solubility of a salt in the solvent and easy handling of the mineral acid. Anion exchange resins, particularly basic anion exchange resins are preferable as the ion exchange resin. Particularly preferred ion exchange resins are resins having an amino functional group. Examples of the ion exchange resin are basic anion exchange resins whose functional group is a dimethylamino group, a 1-deoxy-1-(methylamino)glycitol group or the like. Commercially available examples of such ion exchange resins are "XE-583", "IRA-743", "IRA-96SB" and "XT6050RF" manufactured by Organo Co., Ltd. and the like. Examples of the method of treating the reduction product with the ion exchange resin are a method wherein a liquid containing the product is flowed through a column filled with the ion exchange resin, a method wherein the ion exchange resin is put in the liquid containing the product and the whole is stirred, and the like. Residual boron or boron compounds can be removed by the ion exchange resin treatment. This makes it possible to inhibit polymeric by-products from forming when the product is purified later by distillation, and the yield increases.

The reduction product can be treated in the same manner as mentioned above with silica gel instead of the ion exchange resin. A high-purity object product can also be obtained by the silica gel treatment for the same reason as that of the ion exchange resin treatment.

### Best Mode for Carrying out the Invention

The present invention is described specifically by Examples hereinafter, but the present invention is not limited to these Examples.

### Example 1: Preparation of 1, 2, 4-butanetriol

i) In 250 ml of ethanol was dissolved 38 g (0.20 mol) of diethyl malate, and 17.0 g (0.45 mol) of sodium borohydride was added thereto while keeping temperature at 20°C.
ii) This mixture was stirred at 20°C for 15 hours and then cooled with ice. To the reaction mixture was added 100 ml of saturated HCl-EtOH, and then the resulting insoluble matter was filtered out. The filtrate was passed through a column filled with 100 g of an ion exchange resin ("XE-583" manufactured by Organo Co., Ltd.) to remove residual boron and then concentrated under reduced pressure. The concentrate was further purified by distillation to give 16.9 g (yield 79.7%) of 1, 2, 4-butanetriol.

### Example 2: Preparation of (S)-1, 2, 4-butanetriol

i) In 250 ml of ethanol was dissolved 38 g (0.20 mol) of diethyl (S)-malate, and 17.0 g (0.45 mol) of sodium borohydride was added thereto while keeping temperature at 20°C.
ii) This mixture was stirred at 20°C for 15 hours and then cooled with ice. To the reaction mixture was added 100 ml of saturated HCl-EtOH, and then the resulting insoluble matter was filtered out. The filtrate was passed through a column filled with 100 g of an ion exchange resin ("XE-583" manufactured by Organo Co., Ltd.) to remove residual boron and then concentrated under reduced pressure. The concentrate was further purified by distillation to give 17.2 g (yield 81.1%, 99.7%ee) of 1, 2, 4-butanetriol.

### Example 3: Preparation of (S)-1, 2, 4-butanetriol

The same procedure as in Example 2 was repeated except that the ion exchange resin was replaced with "IRA-743" manufactured by Organo Co., Ltd. at the step ii) to give 16.7 g (yield 78.8%, 99.6%ee) of (S)-1, 2, 4-butanetriol.

### Example 4: Preparation of (S)-1, 2, 4-butanetriol

The same procedure as in Example 2 was repeated except that saturated HCl-EtOH was replaced with 15 ml (0.28 mol) of sulfuric acid at the step ii) to give 14.8 g (yield 74.5%, 99.7%ee) of (S)-1, 2, 4-butanetriol.

### Example 5: Preparation of (S)-1, 2, 4-butanetriol

The same procedure as in Example 2 was repeated except that the ion exchange resin was replaced with "IRA-96SB" manufactured by Organo Co., Ltd. at the step ii) to give 15.8 g (yield 74.5%, 99.6%ee) of (S)-1, 2, 4-butanetriol.

### Example 6: Preparation of (R)-1, 2, 4-butanetriol

In 250 ml of a mixed solvent of ethanol:toluene=87:13 was dissolved 38 g (0.20 mol) of diethyl (R)-malate, and 17.0 g (0.45 mol) of sodium borohydride was added thereto while keeping temperature at 20°C.

Then, the same procedure as at the step ii) in Example 2 was repeated to give 16.9 g (yield 79.7%, 99.7%ee) of (R)-1, 2, 4-butanetriol.

### Example 7: Preparation of (R)-1, 2, 4-butanetriol

In 250 ml of a mixed solvent of ethanol:isopropanol=87:13 was dissolved 38 g (0.20 mol) of diethyl (R)-malate, and 17.0 g (0.45 mol) of sodium borohydride was added thereto while keeping temperature at 20°C.

Then, the same procedure as at the step ii) in Example 2 was repeated to give 16.5 g (yield 77.8%, 99.7%ee) of (R)-1, 2, 4-butanetriol. Example 8: Preparation of (S)-1, 2, 4-butanetriol

In 250 ml of ethylene glycol was dissolved 38 g (0.20 mol) of diethyl (S)-malate, and 17.0 g (0.45 mol) of sodium borohydride was added thereto while keeping temperature at 20°C.

Then, the same procedure as at the step ii) in Example 2 was repeated to give 15.1 g (yield 70.7%, 99.4%ee) of (S)-1, 2, 4-butanetriol.

### Example 9: Preparation of (S)-1, 2, 4-butanetriol

In 250 ml of propylene glycol was dissolved 38 g (0.20 mol) of diethyl (S)-malate, and 17.0 g (0.45 mol) of sodium borohydride was added thereto while keeping temperature at 20°C.

Then, the same procedure as at the step ii) in Example 2 was repeated to give 13.7 g (yield 64.6%, 99.5%ee) of (S)-1, 2, 4-butanetriol.

### Example 10: Preparation of (S)-1, 2, 4-butanetriol

In 250 ml of ethanol was dissolved 17.2 g (0.20 mol, 95.1%ee) of (S)-3-hydroxy- γ -butyrolactone, and 8.5 g (0.23 mol) of sodium borohydride was added thereto while keeping temperature at 20°C.

Then, the same procedure as at the step ii) in Example 2 was repeated to give 15.2 g (yield 71.7%, 94.8%ee) of (S)-1, 2, 4-butanetriol.

### Example 11: Preparation of (S)-1, 2, 4-butanetriol

In 250 ml of a mixed solvent of ethanol:toluene=87:13 was dissolved 17.2 g (0.20 mol, 95.1%ee) of (S)-3-hydroxy-γ-butyrolactone, and 8.5 g (0.23 mol) of sodium borohydride was added thereto while keeping temperature at 20°C.

Then, the same procedure as at the step ii) in Example 2 was repeated to give 14.8 g (yield 69.8%, 94.7%ee) of (S)-1, 2, 4-butanetriol.

### Example 12: Preparation of (R)-1, 2, 4-butanetriol

In 250 ml of ethanol was dissolved 29.6 g (0.20 mol, 94.4%ee) of ethyl (R)-3, 4-dihydroxybutanoate, and 8.5 g (0.23 mol) of sodium borohydride was added thereto while keeping temperature at 20°C.

Then, the same procedure as at the step ii) in Example 2 was repeated to give 16.8 g (yield 79.2%, 94.1%ee) of (R)-1, 2, 4-butanetriol.

### Example 13: Preparation of (R)-1, 2, 4-butanetriol

In 250 ml of a mixed solvent of ethanol:toluene=87:13 was dissolved 29.6 g (0.20 mol, 94.4%ee) of ethyl (R)-3, 4-dihydroxybutanoate, and 8.5 g (0.23 mol) of sodium borohydride was added thereto while keeping temperature at 20°C.

Then, the same procedure as at the step ii) in Example 2 was repeated to give 16.4 g (yield 77.4%, 94.2%ee) of (R)-1, 2, 4-butanetriol.

### Industrial Applicability

A process for preparation according to the present invention is a process wherein 1, 2, 4-butanetriol, which is a useful compound as a synthetic intermediate of medicines, agricultural chemicals and the like, can be obtained safely, easily and inexpensively without causing problems concerning wastewater, and a process which can advantageously be applied to industry.

## Claims

1. A process for preparing 1, 2, 4-butanetriol **characterized in that** a malic diester, 3-hydroxy- γ -butyrolactone or a 3, 4-dihydroxybutanoate represented by the following formulae (I), or a mixture thereof is reduced with sodium borohydride in an alcohol selected from the group consisting of methanol, ethanol, propanol, isopropanol, ethylene glycol, propylene glycol and a mixture thereof or a mixed solvent consisting of an alcohol selected from the group consisting of methanol, ethanol, propanol, isopropanol, ethylene glycol, propylene glycol and of aromatic solvent(s) to give 1, 2, 4-butanetriol represented by the following formula (II). 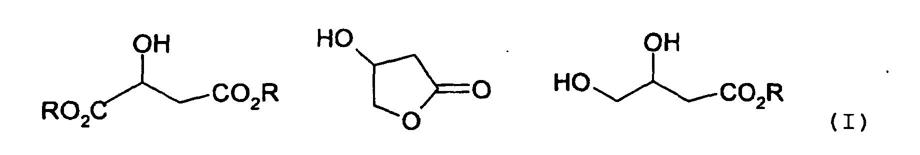 wherein "R" is an alkyl group having four carbon atoms or fewer.

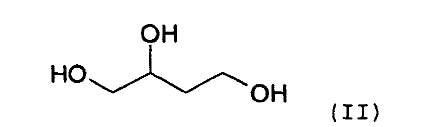

2. A process for preparing 1, 2, 4-butanetriol as claimed in claim 1, wherein the malic diester, 3-hydroxy-γ-butyrolactone or a 3, 4-dihydroxybutanoate are optically active substances represented by the following formulae (III), and 1, 2, 4-butanetriol is an optically active substance represented by the following formula (IV). 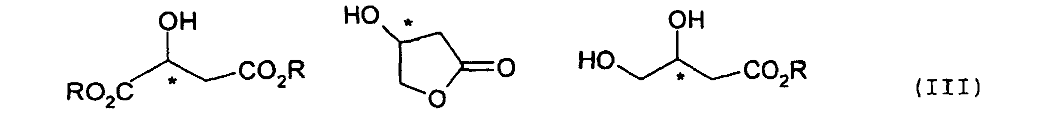 wherein "R" is an alkyl group having four carbon atoms or fewer.

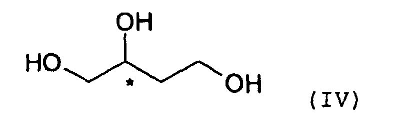

3. A process for preparing 1, 2,4-butanetriol as claimed in claim 1, wherein an equivalent of the alcohol is one to five equivalents to sodium borohydride.

4. A process for preparing 1, 2,4-butanetriol as claimed in claim 1, wherein the alcohol is methanol and/or ethanol.

## Patentansprüche

1. Verfahren zur Herstellung von Verfahren zur Herstellung von 1,2,4-Butantriol, **dadurch gekennzeichnet, dass** ein Äpfelsäurediester, 3-Hydroxy-γ-butyrolacton oder ein 3,4-Dihydroxybutanoat der folgenden Formel (I) oder eine Mischung davon mit Natriumborhydrid in einem Alkohol, der ausgewählt ist aus der Gruppe bestehend aus Methanol, Ethanol, Propanol, Isopropanol, Ethylenglycol, Propylenglycol und einer Mischung davon, oder einem Lösungsmittelgemisch bestehend aus einem Alkohol ausgewählt aus der Gruppe aus Methanol, Ethanol, Propanol, Isopropanol, Ethylenglycol, Propylenglycol und einem oder mehreren aromatischen Lösungsmittel(n) zu 1,2,4-Butantriol der folgenden Formel (II) reduziert wird: 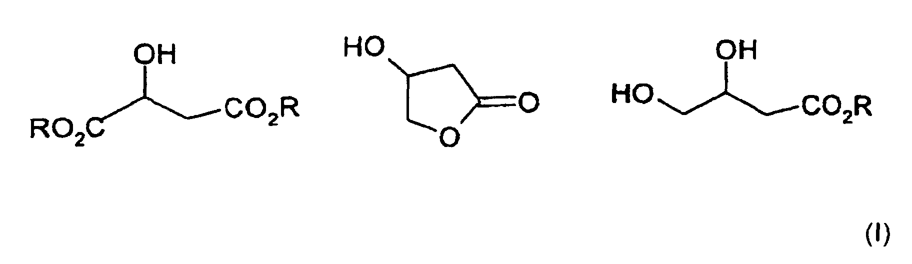 worin "R" für eine Alkylgruppe mit vier oder weniger Kohlenstoffatomen steht.

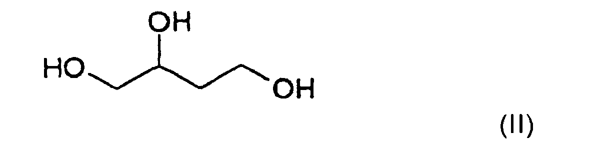

2. Verfahren zur Herstellung von 1,2,4-Butantriol nach Anspruch 1, bei dem der Äpfelsäurediester, 3-Hydroxy-y-butyrolacton oder das 3,4-Dihydroxybutanoat optisch aktive Substanzen der folgenden Formeln (III) sind und es sich bei dem 1,2,4-Butantriol um eine optisch aktive Substanz der folgenden Formel (IV) handelt 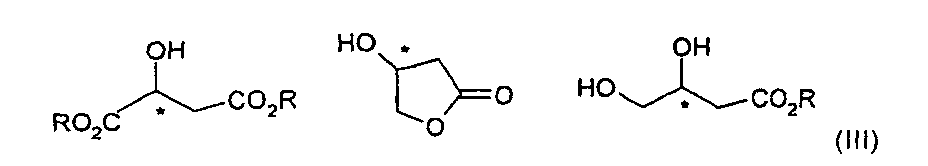 worin "R" eine Alkylgruppe mit vier oder weniger Kohlenstoffatomen bedeutet.

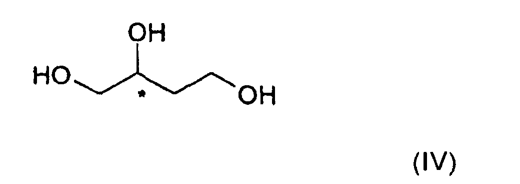

3. Verfahren zur Herstellung von 1,2,4-Butantriol nach Anspruch 1, bei dem für ein Äquivalent des Alkohols ein bis fünf Äquivalent(e) Natriumborhydrid eingesetzt werden.

4. Verfahren zur Herstellung von 1,2,4-Butantriol nach Anspruch 1, bei dem als Alkohol Methanol und/oder Ethanol eingesetzt wird.

## Revendications

1. Procédé de préparation du 1,2,4-butanetriol, caractérisé en que qu'un diester malique, la 3-hydroxy--butyrolactone ou un 3,4-dihydroxybutanoate représentés par les formules (I) suivantes 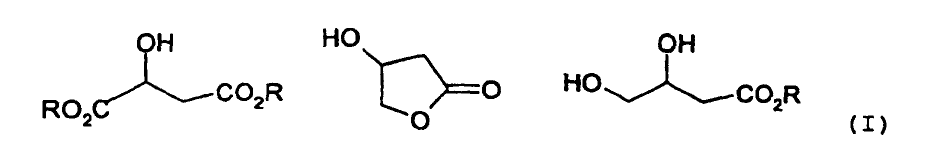 où "R" représente un groupe alkyle ayant quatre atomes de carbone ou moins.
ou un de leurs mélanges, est réduit avec du borohydrure de sodium dans un alcool choisi dans le groupe constitué du méthanol, de l'éthanol, du propanol, de l'isopropanol, de l'éthylèneglycol, du propylèneglycol et d'un de leurs mélanges, ou dans un solvant constitué d'un alcool choisi dans le groupe constitué du méthanol, de l'éthanol, du propanol, de l'isopropanol, de l'éthylèneglycol, du propylèneglycol et d'un ou plusieurs solvants aromatiques pour donner le 1,2,4-butanetriol représenté par la formule (II) suivante

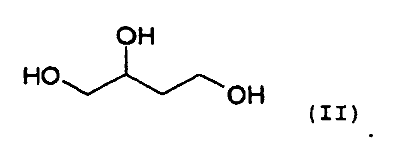

2. Procédé de préparation du 1,2,4-butanetriol selon la revendication 1, dans lequel le diester malique, la 3-hydroxy--butyrolactone ou un 3,4-dihydroxybutanoate sont des substances optiquement actives représentées par les formules (III) suivantes 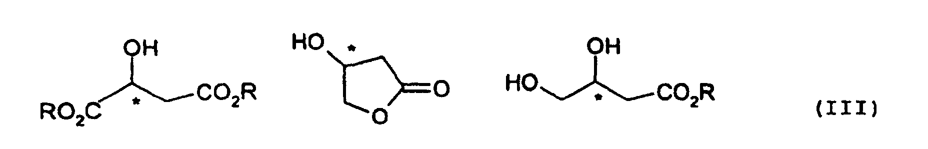 où "R" représente un groupe alkyle ayant quatre atomes de carbone ou moins,
et le 1,2,4-butanetriol est une substance optiquement active représentée par la formule (IV) suivante

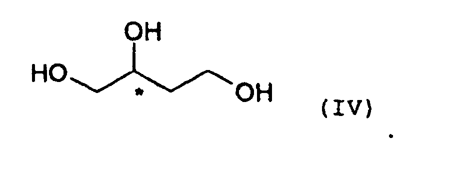

3. Procédé de préparation du 1,2,4-butanetriol selon la revendication 1, dans lequel on utilise un à cinq équivalents d'alcool pour un équivalent de borohydrure de sodium.

4. Procédé de préparation du 1,2,4-butanetriol selon la revendication 1, dans lequel l'alcool est le méthanol et/ou l'éthanol.
